# EUROPEAN PATENT APPLICATION

(11) **EP 4 534 006 A1**
(43) Date of publication of application: **09.04.2025**
(21) Application number: 23201703.8
(22) Date of filing: 04.10.2023
(51) Int. Cl.: A61B 5/11, A61B 5/00

(54) **SYSTEMS AND METHODS OF IMPROVING WALKING SPEED AND DISTANCE ESTIMATION BY FUSION OF CO-LOCATED ACTIVITY MONITORING DEVICES**

(71) Applicant: Koninklijke Philips N.V., 5656 AG Eindhoven (NL)
(72) Inventor: COX, Lieke Gertruda Elisabeth, 5656 AG Eindhoven (NL)
(74) Representative: Philips Intellectual Property & Standards

(57) **Abstract**

The present disclosure is directed to systems and methods adapted for improving gait tracking of individuals having atypical or anomalous walking patterns through the fusion of co-located activity monitoring devices. As described herein, hospital staff and patients can be equipped with activity monitoring devices that are used by the systems and methods to detect proximity of such devices and subsequently update the parameters used to track the gait of the patient. For example, once two or more devices are detected in close proximity (e.g. nurse walking with patient), the estimations from both devices can be compared. Subsequently, estimations for both can be updated by tuning model parameters, e.g., by giving qual weight to both estimations so tuning to reach the average speed, or by weighted tuning based on reliability parameters, that indicate the reliability of individual estimations.

## Description

### FIELD OF THE DISCLOSURE

The present disclosure relates generally to systems and methods of monitoring ambulatory patients, and more specifically to systems and methods of improving monitoring of gait metrics for patients having atypical gaits by fusion of co-located activity monitoring devices.

### BACKGROUND

Gait metrics such as walking speed and distance can be important for estimating patient condition and/or changes in patient condition. Algorithms exist to estimate walking speed, e.g. from accelerometer data from wearable devices. Such algorithms may work well for certain types of individuals, but can have a bias or be less accurate for others, especially patients who have atypical or anomalous walking patterns (e.g., due to the use of walking aids, etc.). Further, such gait estimations may be improved in some situations (e.g., while outdoors) by calibrating or personalizing monitoring algorithms using GPS data, but this approach is less reliable / accurate in an indoor hospital- or clinical-type setting.

### SUMMARY OF THE DISCLOSURE

The present disclosure is directed to systems and methods adapted for improving gait tracking of individuals having atypical or anomalous walking patterns through the fusion of co-located activity monitoring devices. As described herein, hospital staff and patients can be equipped with activity monitoring devices that are used by the systems and methods to detect proximity of such devices and subsequently update the parameters used to track the gait of the patient. For example, once two or more devices are detected in close proximity (e.g. nurse walking with patient), the estimations from both devices can be compared. Subsequently, estimations for both can be updated by tuning model parameters, e.g., by giving qual weight to both estimations so tuning to reach the average speed, or by weighted tuning based on reliability parameters, that indicate the reliability of individual estimations.

According to an embodiment of the present disclosure, a patient monitoring system configured to measure one or more gait metrics associated with a subject is provided. The system can include: a computer-readable storage medium having stored thereon machine-readable instructions to be executed by one or more processors; and one or more processors configured by the machine-readable instructions stored on the computer-readable storage medium to perform the following: (i) detect when a subject device and an assistant device are within a predetermined proximity with one another; (ii) determine whether the subject device and the assistant device remain within the predetermined proximity for at least one of a predetermined time and/or a predetermined distance; (iii) obtain subject data for a first gait metric associated with a subject from the subject device; (iv) obtain assistant data for the first gait metric associated with an assistant from the assistant device; and (v) estimate a true gait metric for the subject based on the subject data and the assistant data obtained via the assistant device and the subject device.

In an aspect, the patient monitoring system can further include: a subject device configured to be worn by the subject, the subject device comprising at least a first sensor configured to measure a gait metric for the subject.

In an aspect, the subject device may comprise the computer-readable storage medium and the one or more processors.

In an aspect, the patient monitoring system can further include: an assistant device configured to be worn by an assistant, the assistant device comprising at least a second sensor configured to measure a gait metric for the assistant.

In an aspect, the assistant device may comprise the computer-readable storage medium and the one or more processors.

According to another embodiment of the present disclosure, a non-transitory computer-readable storage medium having stored thereon machine-readable instructions is provided. When executed by one or more processors, the machine-readable instructions cause the one or more processors to perform operations comprising: detecting when a subject device and an assistance device are within a predetermined proximity with one another; determining whether the subject device and the assistant device remain within the predetermined proximity for at least one of a predetermined time and/or a predetermined distance; obtaining subject data for a first gait metric associated with a subject from the subject device; obtaining assistant data for the first gait metric associated with an assistant from the assistant device; and estimating a true gait metric for the subject based on the subject data and the assistant data obtained via the subject device and the assistant device.

According to yet another embodiment of the present disclosure, a computer-implemented method for measuring one or more gait metrics associated with a subject is provided. The method may include: detecting when a subject device and an assistance device are within a predetermined proximity with one another; determining whether the subject device and the assistant device remain within the predetermined proximity for at least one of a predetermined time and/or a predetermined distance; obtaining subject data for a first gait metric associated with a subject from the subject device; obtaining assistant data for the first gait metric associated with an assistant from the assistant device; and estimating a true gait metric for the subject based on the subject data and the assistant data obtained via the subject device and the assistant device.

These and other aspects of the various embodiments will be apparent from and elucidated with reference to the embodiments described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the various embodiments.
FIG. 1 is a diagram illustrating the use of a patient monitoring system in accordance with certain aspects of the present disclosure.
FIG. 2A is a chart illustrating the fusion of co-located activity monitoring devices in accordance with aspects of the present disclosure.
FIG. 2B is another chart illustrating the fusion of co-located activity monitoring devices in accordance with further aspects of the present disclosure.
FIG. 3 is a flowchart illustrating a method for measuring gait metrics associated with a subject in accordance with certain aspects of the present disclosure.

### DETAILED DESCRIPTION OF EMBODIMENTS

The present disclosure is directed to systems and methods adapted to improve gait tracking for individuals having atypical or anomalous walking patterns. As described herein measuring gait metrics such as walking speed and distance can be important for estimating patient condition and/or changes in patient condition. Although various algorithms exist to estimate walking speed (e.g., from accelerometer data from wearable devices), such algorithms do not work well for patients that have atypical or anomalous walking patterns (e.g., due to the use of walking aids, etc.). Furthermore, conventional approaches to increasing sensitivity and/or accuracy of gait tracking that use GPS data have limited application in an indoor hospital or clinical setting. Thus, the systems and methods of the present disclosure utilize the fusion of co-located activity monitoring devices to improve and/or enhance gait tracking of individuals with atypical or anomalous walking patterns without the use of GPS tracking.

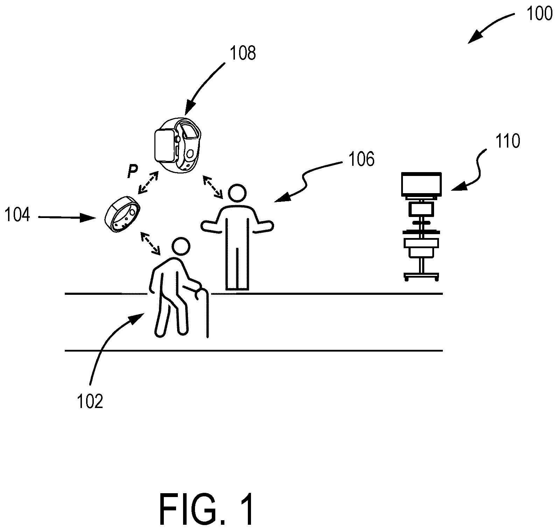

With reference to FIG. 1, a diagram illustrating the operation of a patient monitoring system 100 configured to measure one or more gait metrics associated with a subject 102 is shown in accordance with various aspects of the present disclosure. As described herein, the subject 102 can be a patient having an atypical or anomalous walking pattern due to, for example, the use of a walking aid, an injury affecting the patient's gait, and/or the like. The subject 102 may be equipped with one or more activity monitoring devices, such as activity monitoring device 104. Similarly, one or more other individuals, such as individual 106, may also be equipped with one or more activity monitoring devices, such as activity monitoring device 108.

In embodiments, the activity monitoring devices 104, 108 can be configured to be worn by the subject 102 and/or another individual 106. That is, the devices 104, 108 may be wearable devices. In particular embodiments, each device 104, 108 can comprise at least one sensor 112, 114 configured to measure a gait metric for the corresponding individual 102, 106. For example, each device 104, 108 may include one or more accelerometers, barometers, photoplethysmography sensors, and/or the like. In specific embodiments, the gait metric measured by the devices 104, 108 can include, but is not limited to, walking speed and/or walking distance.

The patient monitoring system 100 can be configured to detect when one or more activity monitoring devices 108 come within a predetermined proximity P of the activity monitoring device 104 worn by the subject 102. As described herein, hospital staff 106 and patients 102 can be equipped with activity monitoring devices 104, 108 that are used by the system 100 to detect proximity of such devices 104, 108 and subsequently update the parameters used to track the gait of the patient 102. For example, in particular embodiments, the individual 106 may be a hospital staff member such as a physical therapist that is assisting the patient 102 to undergo physical therapy, during which it is valuable to measure certain gait metrics of the patient 102.

As described herein, the individuals 106 assisting the patient 102 may be referred to as an assistant 106, and the activity monitoring devices 108 worn by the assistants 106 may be referred to as an assistant device 108. Because a variety of hospital staff and/or other healthcare professionals may be in a position to assist many different patients 102, each of these individuals 106 may be equipped with an assistant device 108.

In embodiments, the proximity P may be predetermined based on the type of activity being monitored or set globally for all devices 104, 106. The proximity P may be predetermined such that it is indicative of an assistant 106 (such as a hospital staff member, etc.) assisting a patient 102. In some embodiments, the proximity P may be preset to be about 2 meters, including less than about 2 meters.

In embodiments, once two or more devices 104, 108 are detected in close proximity P, the system 100 may obtain gait estimation data from both the devices 104, 108 and compare such data to improve the accuracy and/or reliability of the tracking associated with the subject 102.

In some embodiments, the system 100 may be configured to determine whether the subject device 104 and the assistant device 108 remain within the predetermined proximity P for at least a predetermined time and/or a predetermined distance before using data from both devices 104, 108 as described herein. For example, if an assistant device 108 only remains within the proximity P of the subject device 102 for a short period of time, this may indicate that the assistant 106 was just passing by the subject 102 and should not be used for gait metric tracking.

In some embodiments, the system 100 may obtain subject data for one or more gait metrics associated with the subject 102 from the subject device 104, and may obtain assistant data for one or more gait metrics associated with the assistant 106 from the assistant device 108. In embodiments, the system 100 can obtain subject data and assistant data for at least one common gait metric associated with the subject 102 and the assistant 106, respectively. For example, in some embodiments, the system 100 may obtain subject data for a first gait metric associated with a subject 102 from the subject device 104, and obtain assistant data for the first gait metric associated with an assistant 106 from the assistant device 108.

In embodiments, once the subject data and assistant data have been obtained, a true gait metric (e.g., walking speed, walking distance) may be estimated based thereon. For example, in particular embodiments, the algorithms and/or parameters used by the subject device 104 to determine the gait metric may be updated based on the assistant data obtained from the assistant device 108.

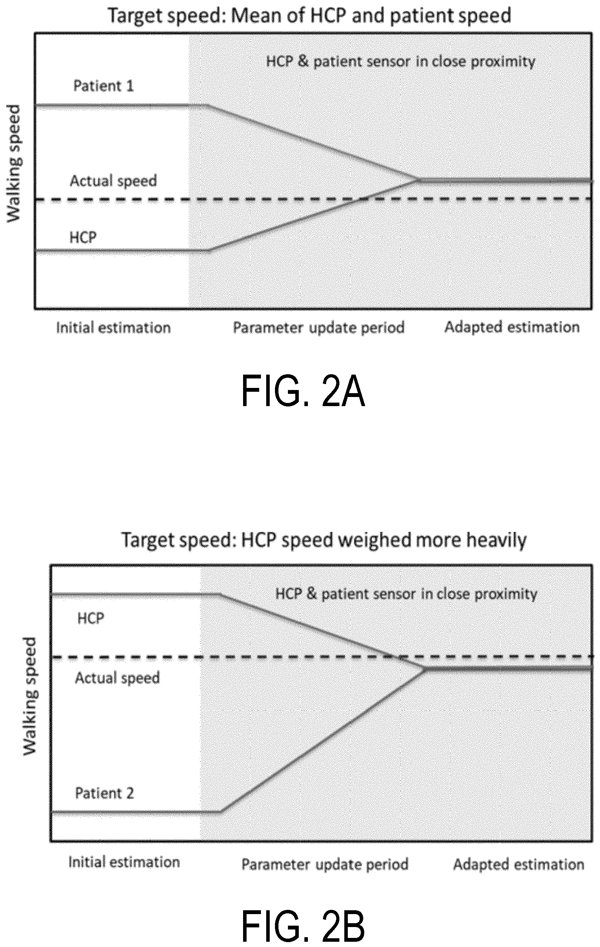

In some embodiments, a reliability score per output may optionally determine a weighing factor of individual estimations. For example, as shown in FIG. 2A, the walking speed of a healthcare professional HCP and a patient PATIENT 1 are separately estimated using an assistant device 108 and a subject device 104, respectively. Neither estimation is absolutely accurate, however, by averaging the estimations, a value closer to the actual speed of the patient 102 can be determined. In some embodiments, a weighted result may be obtained by assigning a higher reliability value to the assistant data (i.e., the data associated with healthcare professional HCP).

For example, once two or more devices are detected in close proximity (e.g. nurse walking with patient), the estimations from both devices can be compared. Subsequently, estimations for both can be updated by tuning model parameters, e.g., by giving qual weight to both estimations so tuning to reach the average speed, or by weighted tuning based on reliability parameters, that indicate the reliability of individual estimations.

It should be appreciated that the weight of the tuning may be adjusted based on the reliability parameter such that, for example, the reliability score depends on the role of the individual 102, 106 wearing the device 104, 108. Additionally, it should be appreciated that reliability scores may improve over time, such as after multiple calibrations. In embodiments, different reliability scores may exist for different walking speeds (e.g., an algorithm has become accurate for slow walking for a nurse, while it has not been updated for faster walking). In further embodiments, if an individual 102, 106 wears the device 104, 108 outside of the hospital and additional tracking information (e.g., GPS information) is available, such information may be used to tune the other algorithms used to estimate gait metrics in accordance with the present disclosure.

In embodiments, it is contemplated that the algorithms used by the devices 104, 108 to determine a gait metric may be the same or may be different for each individual 102, 106. That is, each device 104, 108 and each individual 102, 106 may have a different algorithm running. In some embodiments, a switch may be made to a different algorithm giving results closer to the actual speed based on the subject data and assistant data obtained.

In embodiments, instead of completely moving towards the same actual speed, a maximum update threshold may be set in order to avoid overcorrection (i.e., the true gait metric cannot be more than a certain percentage difference from the observed value).

As described herein, the system 100 can comprise a computer-readable storage medium having stored thereon machine-readable instructions to be executed by one or more processors; and one or more processors configured by the machine-readable instructions stored on the computer-readable storage medium to perform one or more of the operations described above. In particular embodiments, the subject device 104 performs one or more of the operations described above, i.e., the subject device 104 comprises the computer-readable storage medium and the one or more processors. In further embodiments, the assistant device 108 performs one or more of the operations described above, i.e., the assistant device 108 comprises the computer-readable storage medium and the one or more processors. In still further embodiments, an external device 110 (e.g., a patient monitor) can be configured to perform one or more of the operations described above, i.e., the external device 110 comprises the computer-readable storage medium and the one or more processors.

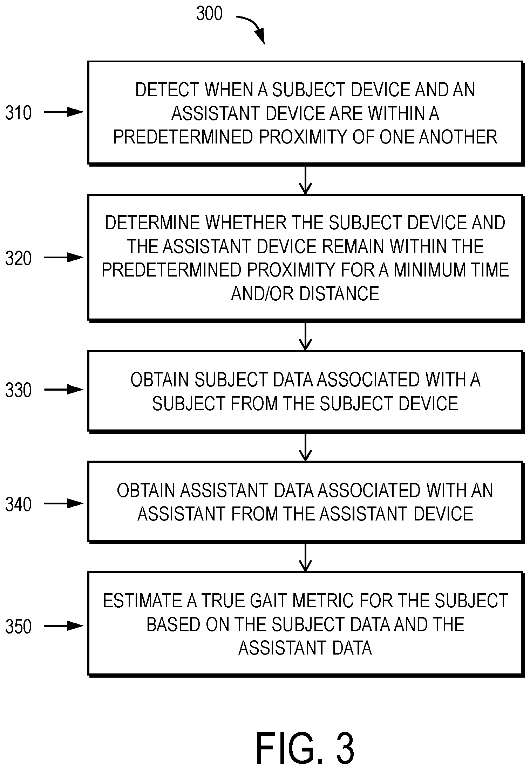

With reference to FIG. 3, a computer-implemented method 300 for measuring one or more gait metrics associated with a subject 102 is provided. As shown, the method 300 comprises: in a step 310, detecting when a subject device and an assistance device are within a predetermined proximity with one another; in a step 320, determining whether the subject device and the assistant device remain within the predetermined proximity for at least one of a predetermined time and/or a predetermined distance; in a step 330, obtaining subject data for a first gait metric associated with a subject from the subject device; in a step 340, obtaining assistant data for the first gait metric associated with an assistant from the assistant device; and in a step 350, estimating a true gait metric for the subject based on the subject data and the assistant data obtained via the subject device and the assistant device.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

As used herein, although the terms first, second, third, etc. may be used herein to describe various elements or components, these elements or components should not be limited by these terms. These terms are only used to distinguish one element or component from another element or component. Thus, a first element or component discussed below could be termed a second element or component without departing from the teachings of the inventive concept.

Unless otherwise noted, when an element or component is said to be "connected to," "coupled to," or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of' and "consisting essentially of' shall be closed or semi-closed transitional phrases, respectively.

It should also be understood that, unless clearly indicated to the contrary, in any methods claimed herein that include more than one step or act, the order of the steps or acts of the method is not necessarily limited to the order in which the steps or acts of the method are recited.

The above-described examples of the described subject matter can be implemented in any of numerous ways. For example, some aspects can be implemented using hardware, software or a combination thereof. When any aspect is implemented at least in part in software, the software code can be executed on any suitable processor or collection of processors, whether provided in a single device or computer or distributed among multiple devices/computers.

The present disclosure can be implemented as a system, a method, and/or a computer program product at any possible technical detail level of integration. The computer program product can include a computer readable storage medium (or media) having computer readable program instructions thereon for causing a processor to carry out aspects of the present disclosure.

The computer readable storage medium can be a tangible device that can retain and store instructions for use by an instruction execution device. The computer readable storage medium can be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium comprises the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network can comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present disclosure can be assembler instructions, instruction-set-architecture (ISA) instructions, machine instructions, machine dependent instructions, microcode, firmware instructions, state-setting data, configuration data for integrated circuitry, or either source code or object code written in any combination of one or more programming languages, comprising an object oriented programming language such as C++, or the like, and procedural programming languages, such as the "C" programming language or similar programming languages. The computer readable program instructions can execute entirely on the user's computer, partly on the user's computer, as a stand-alone software package, partly on the user's computer and partly on a remote computer or entirely on the remote computer or server. In the latter scenario, the remote computer can be connected to the user's computer through any type of network, comprising a local area network (LAN) or a wide area network (WAN), or the connection can be made to an external computer (for example, through the Internet using an Internet Service Provider). In some examples, electronic circuitry comprising, for example, programmable logic circuitry, field-programmable gate arrays (FPGA), or programmable logic arrays (PLA) can execute the computer readable program instructions by utilizing state information of the computer readable program instructions to personalize the electronic circuitry, in order to perform aspects of the present disclosure.

Aspects of the present disclosure are described herein with reference to flowchart illustrations and/or block diagrams of methods, apparatus (systems), and computer program products according to examples of the disclosure. It will be understood that each block of the flowchart illustrations and/or block diagrams, and combinations of blocks in the flowchart illustrations and/or block diagrams, can be implemented by computer readable program instructions.

The computer readable program instructions can be provided to a processor of a, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions can also be stored in a computer readable storage medium that can direct a computer, a programmable data processing apparatus, and/or other devices to function in a particular manner, such that the computer readable storage medium having instructions stored therein comprises an article of manufacture comprising instructions which implement aspects of the function/act specified in the flowchart and/or block diagram or blocks.

The computer readable program instructions can also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various examples of the present disclosure. In this regard, each block in the flowchart or block diagrams can represent a module, segment, or portion of instructions, which comprises one or more executable instructions for implementing the specified logical function(s). In some alternative implementations, the functions noted in the blocks can occur out of the order noted in the Figures. For example, two blocks shown in succession can, in fact, be executed substantially concurrently, or the blocks can sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Other implementations are within the scope of the following claims and other claims to which the applicant can be entitled.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A patient monitoring system (100) configured to measure one or more gait metrics associated with a subject (102), the system comprising:
a computer-readable storage medium having stored thereon machine-readable instructions to be executed by one or more processors; and
one or more processors configured by the machine-readable instructions stored on the computer-readable storage medium to perform the following: (i) detect when a subject device (104) and an assistant device(108) are within a predetermined proximity with one another; (ii) determine whether the subject device and the assistant device remain within the predetermined proximity for at least one of a predetermined time and/or a predetermined distance; (iii) obtain subject data for a first gait metric associated with a subject from the subject device (104); (iv) obtain assistant data for the first gait metric associated with an assistant (106) from the assistant device (108) ; and (v) estimate a true gait metric for the subject (102) based on the subject data and the assistant data obtained via the assistant device (108) and the subject device (104).

2. The patient monitoring system (100) according to claim 1, further comprising:
a subject device (104) configured to be worn by the subject (102), the subject device (104) comprising at least a first sensor (112) configured to measure a gait metric for the subject.

3. The patient monitoring system (100) according to claims 1 or 2, wherein the subject device (104) further comprises the computer-readable storage medium and the one or more processors.

4. The patient monitoring system (100) according to claim 1, further comprising:
an assistant device (108) configured to be worn by an assistant, the assistant device comprising at least a second sensor (114) configured to measure a gait metric for the assistant.

5. The patient monitoring system (100) according to claims 1 or 4, wherein the assistant device (108) further comprises the computer-readable storage medium and the one or more processors.

6. A non-transitory computer-readable storage medium having stored thereon machine-readable instructions that, when executed by one or more processors, cause the one or more processors to perform operations comprising:
detecting when a subject device and an assistance device are within a predetermined proximity with one another;
determining whether the subject device and the assistant device remain within the predetermined proximity for at least one of a predetermined time and/or a predetermined distance;
obtaining subject data for a first gait metric associated with a subject from the subject device;
obtaining assistant data for the first gait metric associated with an assistant from the assistant device; and
estimating a true gait metric for the subject based on the subject data and the assistant data obtained via the subject device and the assistant device.

7. A computer-implemented method (300) for measuring one or more gait metrics associated with a subject, the method comprising:
detecting (310) when a subject device and an assistance device are within a predetermined proximity with one another;
determining (320) whether the subject device and the assistant device remain within the predetermined proximity for at least one of a predetermined time and/or a predetermined distance;
obtaining (330) subject data for a first gait metric associated with a subject from the subject device;
obtaining (340) assistant data for the first gait metric associated with an assistant from the assistant device; and
estimating (350) a true gait metric for the subject based on the subject data and the assistant data obtained via the subject device and the assistant device.
